# EUROPEAN PATENT SPECIFICATION

(11) **EP 2 992 330 B1**
(45) Date of publication and mention of the grant of the patent: **18.04.2018**
(21) Application number: 14720964.7
(22) Date of filing: 29.04.2014
(51) Int. Cl.: G01N 33/574

(54) **METHOD OF DIAGNOSING CANCER**
VERFAHREN ZUR DIAGNOSE VON KREBS
PROCÉDÉ DE DIAGNOSTIC DU CANCER

(30) Priority: 29.04.2013 EP 13165784
(43) Date of publication of application: 09.03.2016
(73) Proprietor: Apogenix AG, 69120 Heidelberg (DE)
(72) Inventor: FRICKE, Harald, 68165 Mannheim (DE); GIEFFERS, Christian, 69221 Dossenheim (DE); SYKORA, Jaromir, 69120 Heidelberg (DE)
(74) Representative: Heubeck, Christian
(86) International application number: PCT/EP2014/058746
(87) International publication number: WO 2014/177576

(56) References cited:
- WO-A2-03/079750
- WO-A2-2008/080623
- WO-A2-2013/022995
- REIMER ET AL: "FasL:Fas ratio--a prognostic factor in breast carcinomas.", CANCER RESEARCH, vol. 60, no. 4, 1 February 2000 (2000-02-01), pages 822-828, XP55065630, ISSN: 0008-5472
- TAC JUNG JANG: "expression of CD40 and Fas ligand in Bowen's disease, Squamous cell carcinoma and Basal cell carcinoma", YONSEI MEDICAL JOURNAL, vol. 43, no. 3, 1 June 2002 (2002-06-01), pages 304-308, XP055129928,
- J.E SIGEL ET AL: "Modern Pathology - Immunohistochemical Analysis of CD30-Positive Lymphoproliferative Disorders for Expression of CD95 and CD95L", MOD PATHOL, vol. 13, no. 4, 1 April 2000 (2000-04-01), pages 446-451, XP055129929,

## Description

The present invention relates to a method for diagnosing glioblastoma, comprising (a) determining the expression of CD95L in a glioblastoma sample, and (b) classifying the glioblastoma disease according the level of CD95L expression.

Another aspect is a method of prognosing the overall survival time or/and the relaps-free survival time in a glioblastoma patient.

A further aspect relates to a CD95L inhibitor for use in the treatment of glioblastoma.

Invasion of surrounding brain tissue by isolated tumor cells represents one of the main obstacles to an effective therapy of glioblastoma multiforme (GBM). Gliomas encompass the majority of tumors originating in the central nervous system (CNS). In adults, the most common tumors are high-grade neoplasms derived from astrocytes or oligodendrocytes. The World Health Organization classifies these malignant tumors according to their degree of anaplasia into grade II (diffuse astrocytoma), grade III (anaplastic astrocytoma) and grade IV (GBM) (Kleihues, P., Burger, P. C., & Scheithauer, B. W. The new WHO classification of brain tumors Brain Pathol. 3, 255-268, 1993). Gliomas account for more than 50% of all brain tumors and are by far the most common primary brain tumors in adults. Despite, development of new diagnostic technologies, the survivalrate is extremely low. Only 3% are still alive five years after diagnosis. The clinical outcome of malignant gliomas depends on the invasion of isolated tumor cells in the nomal brain tissue. Migrating cells can escape the surgical ablation of the tumor and are then the prime targets of post-surgical radiotherapy and adjuvant chemotherapy. Chemotherapeutic agents and irradiation act primarily by inducing apoptosis. This induction of apoptosis often involves activation of the CD95 (Apo-1/Fas) death receptor/ligand system. Nevertheless, most malignant glioma cells are resistant to CD95-induced apoptosis.

WO 2008/080623 describes the treatment of glioblastoma by neutralizing CD95 activity. In this context, CD95L is considered to be expressed in all patients suffering from glioblastoma. This assumption was refuted by the present invention, wherein it was found that glioblastoma diseases can be divided into two groups, i.e. C95L positive and CD95L negative subtypes.

In the present invention, it was surprisingly found that the expression of CD95L in the glioblastoma samples is associated with a reduced survival of the patients. This means that CD95L expression is a prognostic factor of the overall survival time of a glioblastoma patient. Example 1 demonstrates in a clinical phase II study that in tumor samples having the diagnosis "glioblastoma", CD95L is expressed to a variable extent.

Furthermore, Example 1 surprisingly demonstrates that a CD95L inhibitor, for example APG101, can improve the survival of glioblastoma patients suffering from glioblastoma expressing CD95L. In contrast, patients suffering from a glioblastoma which does not express CD95L, do not benefit from the treatment with a CD95L inhibitor. Example 1 enables a therapeutic strategy including a diagnostic step comprising determining the CD95L expression in a glioblastoma sample, and then treating the patient with a CD95L inhibitor if CD95L is expressed. A diagnostic differentiation of glioblastoma by expression of CD95L is enabled. This leads to a new diagnostic classification of glioblastoma by the extent of CD95L expression, leading to an adapted therapy of those patients suffering from a glioblastoma expressing CD95L. This strategy is advantageous, because the CD95L inhibitor is administered to those patients only in which a therapeutic success can be expected. This is not disadvantageous in patients suffering from a glioblastoma not expressing CD95L, because these patients will probably not benefit from a treatment with a CD95L inhibitor.

The problem of the present invention can therefore be seen in the improvement of glioblastoma diagnosis and therapy. The solution provided herein includes a diagnostic differentiation of glioblastoma into a sub-type expressing CD95L, and a sub-type which does not express CD95L. These sub-types have hitherto not been identified, and have not been considered to require a specific therapy based upon the CD95L expression. The solution provided in the present invention includes a specific therapy based upon the diagnosis of the sub-types identified in the present invention.

A first aspect of the present invention is a method for diagnosing glioblastoma, comprising
(a) determining the expression of CD95L in a glioblastoma sample, and
(b) classifying the glioblastoma according the level of CD95L expression, wherein the glioblastoma is classified by the level of CD95L expression into a CD95L positive glioblastoma or a CD95L negative glioblastoma, wherein the glioblastoma is regarded as CD95L positive if at least 5% of the cells in the sample express CD95L and/or if CD95L can be detected on at least 5% of the area of tumor tissue in a glioblastoma tissue sample.

The method can be followed by a step (c), i.e. determining and/or selecting a method of treatment which is suitable for the type of glioblastoma that has been diagnosed and/or carrying out the treatment.

In the present invention, expression of CD95L can be determined by any known suitable method. For example, the CD95L mRNA can be determined. A preferred example of a suitable method is a histological, histochemical or/and immunohistochemical method.

The sample employed in the diagnosis of glioblastoma as described herein can be an archived tumor tissue, for example a biopsy or surgery material embedded in paraffin, which has been obtained in an earlier stage of the disease.

The glioblastoma disease can be classified by the level of CD95L expression into a CD95L positive glioblastoma disease or a CD95L negative glioblastoma disease.

In particular the CD95L positive glioblastoma disease is characterised by a cell expressing CD95L on the cell surface. However, the methods described herein may also be based on the detection of intracellular epitopes of CD95L.

Glioblastoma is regarded as CD95L positive, if at least 5% of the cells in a glioblastoma sample express CD95L. The number of CD95L positive cells can be determined by counting the cells in a microscopic section.

CD95L expression is considered to be absent (CD95L negative) if essentially no cells expressing CD95L can be detected in the tissue sample, or if the sample is a sample which does not fulfil the criteria defined herein for a CD95L positive sample (non-positive sample). In a CD95L negative sample, the number of tumor cells expressing CD95L is below the threshold defined herein for CD95L positive samples, i.e. below 5% of tumor cells.

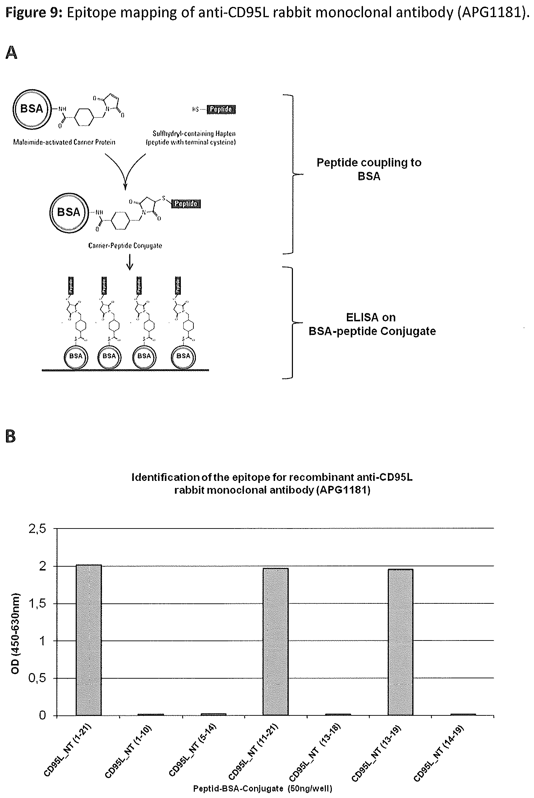

CD95L expression in cells can be determined by histochemical methods, such as the method disclosed in Example 2. In particular the expression of CD95L in the glioblastoma sample can be determined by contacting the sample with an agent specifically binding to CD95L. For example, CD95L inhibitors, as disclosed herein, can be used for determination of CD95L, as these inhibitors can specifically bind to CD95L. Antibodies specifically binding to CD95L can be used. Suitable antibodies can be prepared by known methods. An example of a suitable antibody is given in Examples 2 and 7. Such anti-CD95L-specific antibodies or CD95L-recognising fragments thereof may bind to the extracellular or intracellular domain of CD95L. According to a preferred embodiment, anti-CD95L-specific antibodies or a CD95L-recognising fragment thereof bind to an intracellular epitope of CD95L. According to an especially preferred embodiment the anti-CD95L-specific antibody or CD95L-recognising fragment thereof binds to tyrosine (Y) in N-terminal position 13 of human CD95L. This tyrosine in position 13 is required for defining the specificity of especially preferred antibodies according to the invention. According to especially preferred embodiments an anti-CD95L antibody or a CD95L-recognising fragment thereof recognises an epitope that encodes the N-terminal amino acids 13-19 of human CD95L (see Figure 9).

It is particularly preferred that the antibodies according to the present invention are monoclonal antibodies.

Other suitable agents specifically binding to CD95L include an extracellular receptor domain of CD95, or a functional fragment thereof, for example in a fusion polypeptide further comprising an Fc domain, or a functional fragment thereof. An example of a suitable fusion polypeptide is APG101, as described herein. Suitable labeling and staining methods are known. An example of staining with a streptavidin-coupled alkaline phosphatase binding to a biotinylated secondary antibody is given in Example 2.

Glioblastoma is also regarded as CD95L positive, if CD95L can be detected on at least 5% of the area of tumor tissue in a tissue section. This value is termed herein as "% CD95L positive area of tumor tissue". Non-tumor tissue is excluded in this analysis. A tissue section can be prepared by known methods. Suitable methods for detection of CD95L are described herein. An exemplary method is described in Example 2. An example of determination of the area of CD95L positive tumor tissue is given in Example 3. CD95L expression is considered to be absent (CD95L negative) if essentially no CD95L can be detected in the tissue sample, or if the value of % CD95L positive area of tumor tissue is below the threshold defined for a CD95 positive sample, i.e. below 5% of tumor area.

CD95L expression (e.g. in terms of cell number or surface in a tissue section) can be determined by known methods, for example by methods based upon automatized analysis of tissue sections.

Diagnosis based upon the expression of CD95L is of particular importance for diagnosis and treatment of glioblastoma which hitherto has not been considered to include CD95L expression sub-types, and has not been considered to require a specific therapy adapted to the diagnosed CD95L expression sub-type. The solution provided herein includes a specific therapy based upon the diagnosis of the CD95L expression sub-types identified in the present invention.

The present invention provides a hitherto unknown disease pattern based upon the CD95L expression in glioblastoma cells.

The glioblastoma can be characterised by invasive growth.

The CD95L inhibitor employed in the present invention can comprise a fusion protein comprising at least an extracellular CD95 domain or a functional fragment thereof and at least a Fc domain or a functional fragment thereof. In particular, the fusion protein is selected from APG101, polypeptides having at least 70% identity to APG101 and functional fragments of APG101.

Fusion proteins comprising the extracellular domain of the death receptor CD95 (Apo-1; Fas) fused to an immunoglobulin Fc domain are described in WO 2004/085478. "Fusion protein", as used herein, includes a mixture of fusion protein isoforms, each fusion protein comprising at least an extracellular CD95 domain (APO-1; Fas) or a functional fragment thereof and at least a second domain being an Fc domain or a functional fragment thereof distributing within a pl range of about 4.0 to about 8.5. Accordingly, the extracellular CD95 domain as used herein may be also called "first domain", while the Fc domain may be called "second domain".
The mixture of fusion proteins can be provided in a composition.
The first domain protein is an extracellular CD95 domain, preferably a mammalian extracellular domain, in particular a human protein, i.e. a human extracellular CD95 domain. The first domain, i.e. the extracellular CD95 domain, of the fusion protein preferably comprises the amino acid sequence up to amino acid 170, 171, 172 or 173 of human CD95 (SEQ ID NO. 1). A signal peptide (e.g. position 1-25 of SEQ ID NO: 1) may be present or not. Particularly for therapeutic purposes the use of a human protein is preferred. The fusion protein can comprise one or more first domains which may be the same or different. One first domain, i.e. one extracellular CD95 domain is preferred to be present in the fusion protein.
According to a preferred embodiment, the Fc domain or functional fragment thereof, i.e. the second domain of the fusion protein according to the invention, comprises the CH2 and/or CH3 domain, and optionally at least a part of the hinge region, or a modified immunoglobulin domain derived therefrom. The immunoglobulin domain may be an IgG, IgM, IgD, or IgE immunoglobulin domain or a modified immunoglobulin domain derived, therefrom. Preferably, the second domain comprises at least a portion of a constant IgG immunoglobulin domain. The IgG immunoglobulin domain may be selected from IgG1, IgG2, IgG3 or IgG4 domains or from modified domains therefrom. Preferably, the second domain is a human Fc domain, such as a IgG Fc domain, e.g. a human IgG1 Fc domain.

The fusion protein can comprise one or more second domains which may be the same or different. One second domain, i.e. one Fc domain is preferred to be present in the fusion protein.

Further, both the first and second domains are preferably from the same species.

The first domain, i.e. the extracellular CD95 domain or the functional fragment thereof may be located at the N- or C-terminus. The second domain, i.e. the Fc domain or functional fragment may also be located at the C- or N-terminus of the fusion protein. However, the extracellular CD95 domain at the N-terminus of the fusion protein is preferred.

According to a further preferred embodiment, the fusion protein is APG101 (CD95-Fc, position 26-400 in SEQ ID NO: 1). As defined by SEQ ID NO: 1 APG101 can be a fusion protein comprising a human extracellular CD95 domain (amino acids 26-172) and a human IgG1 Fc domain (amino acids 172-400), further optionally comprising an N-terminal signal sequence (e.g. amino acids 1-25 of SEQ ID NO: 1). The presence of the signal peptide indicates the immature form of APG101. During maturation, the signal peptide is cleaved off. According to an especially preferred embodiment the signal sequence is cleaved off. APG101 with the signal sequence being cleaved off is also comprised by the term "unmodified APG101". In a further embodiment the fusion protein is a polypeptide having at least 70% identity, more preferably 75% identity, 80% identity, 85% identity, 90% identity, 95% identity, 96% identity, 97% identity, 98% identity, 99% identity with APG101. According to the present application the term "identity" relates to the extent to which two amino acid sequences being compared are invariant, in other words share the same amino acids in the same position.

The term "APG101" includes a fusion protein of position 26-400 of SEQ ID NO: 1, with and without a signal peptide. The term "APG101" also includes fusion proteins containing N-terminally truncated forms of the CD95 extracellular domain.
In another preferred embodiment the fusion protein according to the invention is a functional fragment of APG101. As used herein, the term "fragment" generally designates a "functional fragment", i.e. a fragment or portion of a wild-type or full-length protein which has essentially the same biological activity and/or properties as the corresponding wild-type or full-length protein has.
A person skilled in the art is aware of methods to design and produce fusion proteins according to the present invention. The mixture of fusion protein isoforms, in particular APG101 isoforms, however, can be obtained by a method described, e.g., in WO 2004/085478. According to a preferred embodiment designing a fusion protein of the present invention comprises a selection of the terminal amino acid(s) of the first domain and of the second domain in order to create at least one amino acid overlap between both domains. The overlap between the first and the second domain or between the two first domains has a length of preferably 1, 2 or 3 amino acids. More preferably, the overlap has a length of one amino acid. Examples for overlapping amino acids are S, E, K, H, T, P, and D.
As indicated above, "fusion protein", as used herein, includes a mixture of isoforms. The term "isoform" as used herein designates different forms of the same protein, such as different forms of APG101, in particular APG101 without signal sequence. Such isoforms can differ, for example, by protein length, by amino acid, i.e. substitution and/or deletion, and/or post-translational modification when compared to the corresponding unmodified protein, i.e. the protein which is translated and expressed from a given coding sequence without any modification. Different isoforms can be distinguished, for example, by electrophoresis, such as SDS-electrophoresis, and/or isoelectric focussing which is preferred according to the present invention.

Isoforms differing in protein length can be, for example, N- terminally and/or C-terminally extended and/or shortened when compared with the corresponding unmodified protein. For example, a mixture of APG101 isoforms according to the invention can comprise APG101 in unmodified form as well as N- terminally and/or C-terminally extended and/or shortened variants thereof. Thus, according to a preferred embodiment, the mixture according to the invention comprises N-terminally and/or C-terminally shortened variants of APG101. In particular preferred is a mixture of fusion protein isoforms comprising N-terminally shortened fusion proteins. Such N-terminally shortened fusion proteins may comprise -1, -2, -3, -4, -5, -6, -7, -8, -9, -10, -11, -12, -13, -14, -15, -16, -17, -18, -19, -20, -21, -22, -23, -24, -25, - 26, -27, -28, -29, -30, -35, -40, -45 and/or -50 N-terminally shortened variants of unmodified APG101. Particularly preferred are -17, -21 and/or -26 N-terminally shortened variants. The numbering refers to the APG101 protein including signal sequence according to SEQ ID NO: 1. In other words, the shortened fusion proteins can comprise a sequence SEQ ID NO: 1 N-terminally truncated by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45 and/or 50 amino acids. Preferred shortened fusion proteins have SEQ ID NO: 1 N-terminally truncated by 16, 20, or 25 amino acids.

An example for a C-terminal shortening of APG101 isoforms is C-terminal Lys-clipping.

According to a preferred embodiment of the present invention the mixture of fusion proteins according to the present invention preferably comprises 50 mol-% unmodified APG101 in relation to modified isoforms, more preferably 40 mol-% unmodified APG101, more preferably 30 mol-% unmodified APG101, more preferably 20, more preferably 10 mol- % unmodified APG101, more preferably 5 mol-% unmodified APG101 and even more preferably 3 mol-% unmodified APG101 and most preferably 1 mol-% and/or less unmodified APG101. Most preferred is an embodiment comprising a mixture of fusion protein isoforms that does not comprise any unmodified APG101.

As outlined above, isoforms can also differ by amino acid substitution, amino acid deletion and/or addition of amino acids. Such a substitution and/or deletion may comprise one or more amino acids. However, the substitution of a single amino acid is preferred according to this embodiment.

Isoforms according to the invention can also differ with regard to post-translational modification. Post-translational modification according to the present invention may involve, without being limited thereto, the addition of hydrophobic groups, in particular for membrane localisation such as myristoylation, palmitoylation, isoprenylation or glypiation, the addition of cofactors for enhanced enzymatic activity such as lipoyation, the addition of smaller chemical groups such as acylation, formylation, alkylation, methylation, amidation at the C-terminus, amino acid addition, γ-carboxylation, glycosylation, hydroxylation, oxidation, glycilation, biotinylation and/or pegylation.

According to the present invention the addition of sialic acids, Fc-based glycosylation, in particular Fc-based N-terminal glycosylation, and/or pyro-Glu-modification are preferred embodiments of post-translational modification.

According to a preferred embodiment the fusion proteins comprised by the composition of the invention comprise high amounts of sialic acids. According to the present invention the content of sialic acid is preferably from about 4.0 to 7.0 mol NeuAc/mol APG101, more preferably from 4.5 to 6.0 mol NeuAc/mol APG101 and most preferably about 5.0 mol NeuAc/mol APG101. As used herein, sialic acids refer N- or O-substituted derivatives of neuraminic acid. A preferred sialic acid is N-acetylneuraminic acid (NeuAc). The amino group generally bears either an acetyl or glycolyl group but other modifications have been described. The hydroxyl substituents may vary considerably. Preferred hydroxyl substituents are acetyl, lactyl, methyl, sulfate and/or phosphate groups. The addition of sialic acid results generally in more anionic proteins. The resulting negative charge gives this modification the ability to change a protein's surface charge and binding ability. High amounts of sialic acid lead to better serum stability and thus, improved pharmacokinetics and lower immunogenicity. The high degree of sialylation of APG101 isoforms of the present invention could be explained by the high amount of diantennary structure. It has to be regarded as highly surprising that the APG101 isoforms in the composition of the invention obtained by the inventive method show such a high grade of sialic acid addition.

According to the present invention, glycosylation designates a reaction in which a carbohydrate is attached to a functional group of a fusion protein, functional fragment thereof as defined herein. In particular, it relates to the addition of a carbohydrate to APG101 or an isoform thereof. The carbohydrate may be added, for example, by N-linkage or O-linkage. N-linked carbohydrates are attached to a nitrogen of asparagine or arginine site chains. O-linked carbohydrates are attached to the hydroxy oxygen of serine, threonine, tyrosine, hydroxylysine or hydroxyproline side chains. According to the present invention, N-linkage, in particular Fc-based N-terminal glycosylation is preferred. Particularly preferred N-linked glycosylation sites are located at positions N118, N136 and/or N250 of APG101 (SEQ ID NO: 1).

Fucosylation according to the present invention relates to the adding of fucose sugar units to a molecule. With regard to the present invention such an addition of a fucose sugar unit to the fusion protein, and in particular to APG101, represents an especially preferred type of glycosylation. A high portion of fucosylated forms leads to a reduced antibody-dependent cellular cytotoxicity (ADCC). Thus, the mixture of fusion protein isoforms is characterised by reduced ADCC, which is beneficial for pharmaceutical and diagnostic applications.

Beside the first and second domain as defined herein, the fusion proteins according to the invention may comprise further domains such as further targeting domains, e.g. single chain antibodies or fragments thereof and/or signal domains. According to a further embodiment, the fusion protein used according to the invention may comprise an N-terminal signal sequence, which allows secretion from a host cell after recombinant expression. The signal sequence may be a signal sequence which is homologous to the first domain of the fusion protein. Alternatively, the signal sequence may also be a heterologous signal sequence. In a different embodiment the fusion protein is free from an additional N-terminal sequence, such as a signal peptide.

The fusion protein as described herein may be an N-terminally blocked fusion protein, which provides a higher stability with regard to N-terminal degradation by proteases, as well as a fusion protein having a free N-terminus, which provides a higher stability with regard to N-terminal degradation by proteases.

Modifications blocking the N-terminus of protein are known to a person skilled in the art. However, a preferred post-translational modification according to the present invention blocking the N-terminus is the pyro-Glu-modification. Pyro-Glu is also termed pyrrolidone carboxylic acid. Pyro-Glu-modification according to the present invention relates to the modification of an N-terminal glutamine by cyclisation of the glutamine via condensation of the α-amino group with a side chain carboxyl group. Modified proteins show an increased half-life. Such a modification can also occur at a glutamate residue. Particularly preferred is a pyro-Glu-modification, i.e. a pyrrolidone carboxylic acid, with regard to the N-terminally shortened fusion protein -26.

A mixture as described herein may comprise 80-99 mol-% N-terminally blocked fusion proteins and/or 1-20 mol-% fusion proteins having a free N-terminus.

According to a further preferred embodiment the mixture as described herein comprises 0.0 to 5.0 mol-%, more preferably 0.0 to 3.0 mol-% and even more preferably 0.0 to 1.0 mol-%, of fusion protein high molecular weight forms such as aggregates. In a preferred embodiment the mixture does not comprise any aggregates of fusion protein isoforms, in particular no dimers or aggregates of APG101. Dimers or aggregates are generally undesired because they have a negative effect on solubility and immunogenicity.

The functional form of APG101 comprises two fusion proteins, as described herein, coupled by disulfide bridges at the hinge region at positions 179 or/and 182 with reference SEQ ID NO:1 of the two molecules. The disulfide bridge may also be formed at position 173 with reference to SEQ ID NO:1 of the two molecules, resulting in an improved stability. If the disulfide bridge at position 173 with reference to SEQ ID NO:1 is not required, the Cys residue at this position can be replaced by another amino acid, or can be deleted.

According to the invention, the mixture of fusion protein isoforms distributes within a pl range of about 4.0 to about 8.5. In a further embodiment the pl range of the mixture of fusion protein isoforms comprised by the composition according to the invention is about 4.5 to about 7.8, more preferably about 5.0 to about 7.5.

The isoelectric point (pl) is defined by the pH-value at which a particular molecule or surface carries no electrical charge. Depending on the pH range of the surrounding medium the amino acids of a protein may carry different positive or negative charges. The sum of all charges of a protein is zero at a specific pH range, its isoelectric point, i.e. the pl value. If a protein molecule in an electric field reaches a point of the medium having this pH value, its electrophorectic mobility diminishes and it remains at this site. A person skilled in the art is familiar with methods for determining the pl value of a given protein, such as isoelectric focussing. The technique is capable of extremely high resolution. Proteins differing by a single charge can be separated and/or fractionated.

Another aspect of the present invention is a method of prognosing the overall survival time or/and the relapse-free survival time in a glioblastoma patient, said method comprising
(a) determining CD95L expression in a glioblastoma sample,
(b) classifying the glioblastoma by the level of CD95L expression into a CD95L positive glioblastoma or a CD95L negative glioblastoma, and
(b) prognosing the survival time or/and the relapse-free survival time of the patient by the level of CD95L expression, wherein the CD95L expression is negatively correlated with the survival time of the patient.

In the present invention, the overall survival time (OS) denotes the chances of staying alive for a group of individuals suffering from glioblastoma. It denotes the percentage of individuals in the group who are likely to be alive after a particular duration of time.

In this aspect of the present invention, a glioblastoma sample obtained from the patient is used. The claimed method does not include the step of obtaining the glioblastoma sample from the patient.

In this aspect, the glioblastoma disease is classified by the level of CD95L expression into a CD95L positive glioblastoma disease or a CD95L negative glioblastoma disease.

In this aspect, an agent specifically binding to CD95L as described herein can be used for determining CD95L expression. The agent can be any CD95L inhibitor as described herein.

In particular, the agent specifically binding to CD95L inhibitor comprises a fusion protein as described herein, comprising at least an extracellular CD95 domain or a functional fragment thereof and at least a Fc domain or a functional fragment thereof. More particular, the fusion protein is selected from APG101, polypeptides having at least 70% identity to APG101 and functional fragments of APG101.

Also described herein is a method of treatment of glioblastoma, said method comprising
(a) determining the expression of CD95L in a glioblastoma sample obtained from a patient, and
(b) administrating a CD95L inhibitor if in step (a), expression of CD95L has been detected in the glioblastoma sample.

Yet another aspect of the present invention is a CD95L inhibitor for use in the treatment of glioblastoma, wherein the CD95L inhibitor comprises a fusion protein comprising at least an extracellular CD95 domain or a functional fragment thereof and at least a Fc domain or a functional fragment thereof, and wherein the glioblastoma disease is a CD95L positive glioblastoma that has been classified as a CD95L positive glioblastoma according to the method of the invention.

Also described herein is the use of a CD95L inhibitor for the manufacture of a medicament for the treatment of glioblastoma, wherein the glioblastoma is a CD95L positive glioblastoma. The CD95L inhibitor may be any CD95L inhibitor as described herein.

The glioblastoma patient to be diagnosed or/and treated as described herein can be a patient with first or second relapse or progression of glioblastoma. The patient may be a patient wherein standard treatment including radiotherapy (e.g. 60Gy) or/and temozolomide has failed in the treatment of glioblastoma. In particular the patient is a candidate for re-irradiation for treatment of glioblastoma.

In the therapeutic uses as described herein, the CD95L inhibitor can be administered systemically, for example by infusion or injection.

The invention is further illustrated by the following Figures and Examples.

### Figure legends

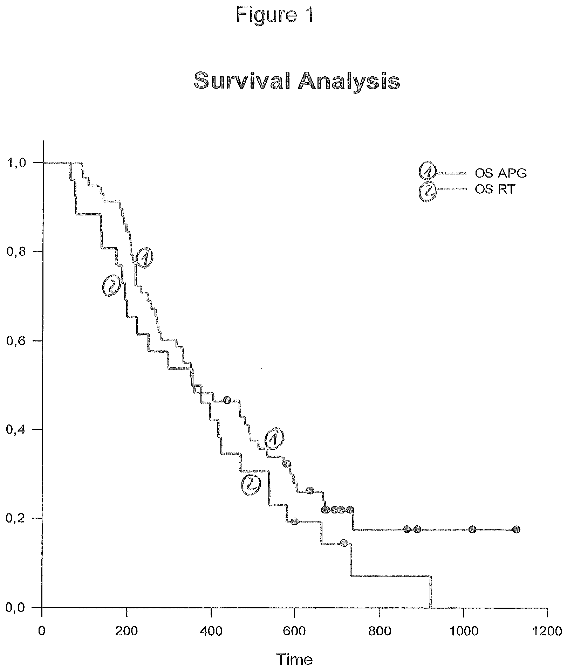

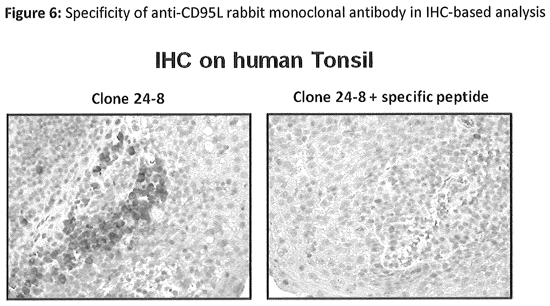

**Figure 1****:** Survival analysis (Kaplan-Meier plot) of GBM patients randomized to the radiation therapy (RT) only group (control group) and the radiation therapy plus APG101 group (test group). (1) Test group. (2) Control group. Abscissa: survival time (days). Ordinate: survival rate. A survival rate of 1.0 indicates 100 % survival.

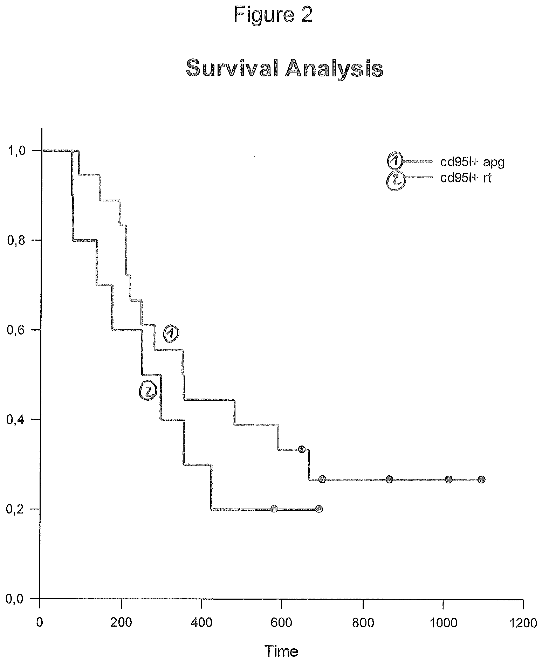

**Figure 2****:** Comparison of the survival data of the subgroup of CD95L positive patients taken from the patients described in Figure 1 (Kaplan-Meier plot). (1) Test group, radiation therapy plus APG101. (2) Control group, radiation therapy only. Abscissa: survival time (days). Ordinate: survival rate. A survival rate of 1.0 indicates 100 % survival.

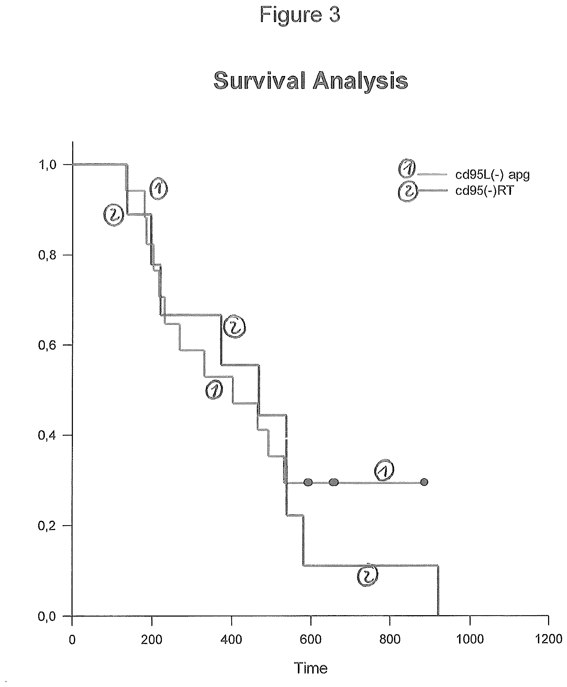

**Figure 3****:** Comparison of the survival data of the subgroup of CD95L negative patients taken from the patients described in Figure 1 (Kaplan-Meier plot). (1) Test group, radiation therapy plus APG101. (2) Control group, radiation therapy only. Abscissa: survival time (days). Ordinate: survival rate. A survival rate of 1.0 indicates 100 % survival.

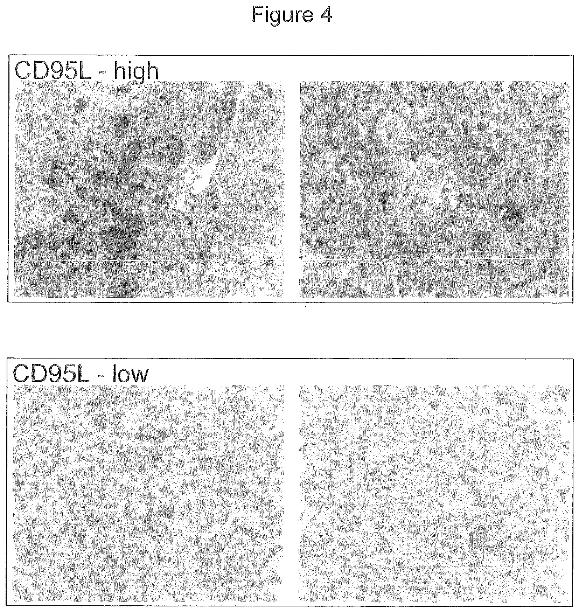

**Figure 4****:** Examples of CD95L expression levels in glioblastoma tissue sections according to Example 3. Examples of high expression (CD95L positive) and low CD95L expression (CD95L negative) are given. The panel shows two examples for each expression level.

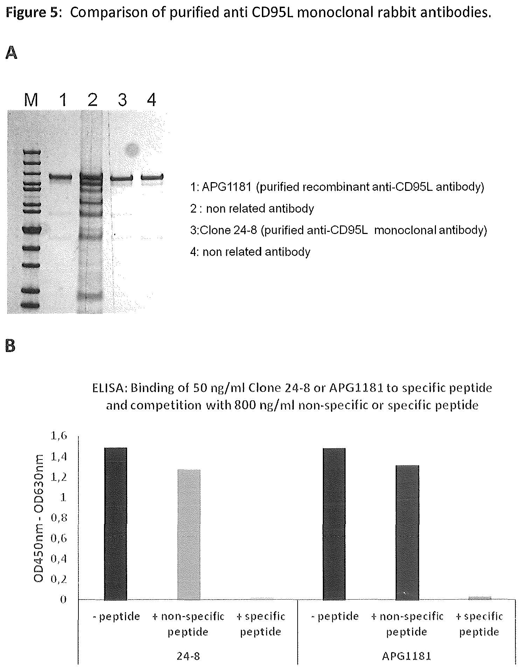

**Figure 5****:** Comparison of purified anti-CD95L monoclonal rabbit antibodies.
   A) Non reduced SDS-Page/Silver staining of purified anti-CD95L rabbit monoclonal antibodies. Lane 1 shows recombinant antibody (APG1181) purified after expression in HEK-cells. Lane 3 shows purified antibody purified form the supernatant of the rabbit hybridoma clone 24-8.
   B) ELISA analysis of purified antibodies: APG1181 and antibody from clone 24-8 were analysed for their specificity to recognise the immobilized CD95L-peptide (CD95L-NT_1-21) used for immunization of the rabbits. Both antibodies specifically bind to the immobilized peptide. The specific recognition for both antibodies was confirmed by competition by the addition of the epitope specific peptide (+ specific peptide). However, an unspecific peptide has no significant competing effect for both antibodies.
      No difference in specificity or sensitivity could be seen between APG1181 and clone 24-8
**Figure 6****:** Specificity of anti-CD95L rabbit monoclonal antibody in IHC-based analysis.
   Human tonsil sections, known to express CD95L in a subset of plasma cells were analysed with the purified antibody from clone 24-8 in the absence or presence of the specific peptide (CD95L-NT_1-21) as indicated. The specific signal seen in the absence (clone 24-8) of the peptide is entirely missing in the presence of the competing peptide confirming the specificity of the antibody.

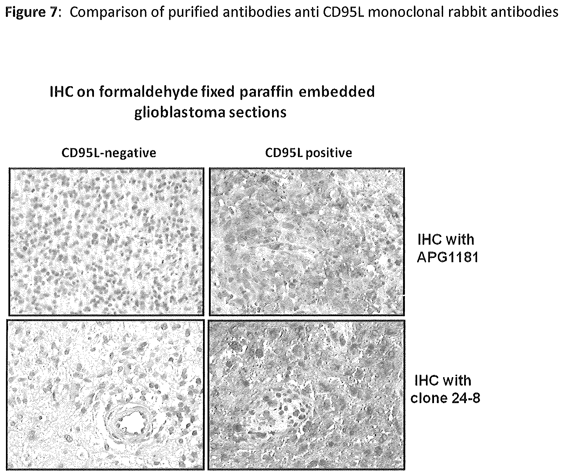

**Figure 7****:** Comparison of purified antibodies anti-CD95L monoclonal rabbit antibodies.
   Purified antibodies APG1181 and antibody from clone 24-8 were analysed for their specificity to recognise CD95L in IHC-analysis on glioblastoma sections. For the analysis two glioblastoma sections were chosen that were previously analysed as CD95L-negative or CD95L-positive, respectively, using a commercial polyclonal anti-CD95L antibody. As seen in the figure no difference in specificity or sensitivity could be seen between APG1181 and clone 24-8 in IHC- based analysis of glioblastoma tissue.

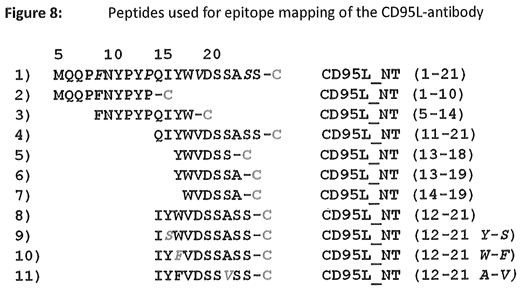

**Figure 8****:** Peptides used for epitope mapping of the CD95L-antibody.
   The peptides shown were used for the identification of the epitope of the anti-CD95L rabbit monoclonal antibody. The peptides encode an N-terminal part of the human CD95L. The first peptide CD95L-NT_1-21) was used for the immunization of rabbits for the generation of rabbit monoclonal antibodies.
   The shorter, truncated versions (peptides 2-7) and the respective permutated peptides (8-11) were used for fine mapping of the epitope of individual antibodies.
**Figure 9****:** Epitope mapping of anti-CD95L rabbit monoclonal antibody (APG1181).
   For the epitope mapping the respective peptides (see figure 8) were coupled to BSA, the peptide-BSA-conjugates (as indicated) were immobilized to an ELISA plate and incubated with the recombinant anti-CD95L rabbit monoclonal antibody (APG1181) followed by a specific peroxidase-conjugated anti-rabbit secondary antibody. As shown in the figure APG1181 specifically recognises an epitope that encodes the N-terminal aa13-19 of human CD95L.

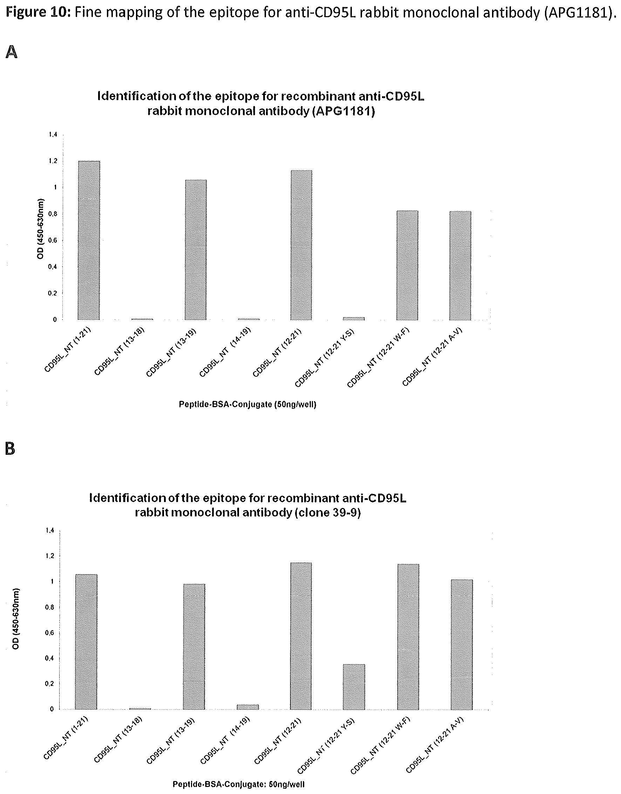

**Figure 10****:** Fine mapping of the epitope for anti-CD95L rabbit monoclonal antibody (APG1181).
   Peptide-BSA-conjugates (as indicated) were immobilized to an ELISA plate and incubated with the recombinant anti-CD95L rabbit monoclonal antibody (APG1181) followed by a specific peroxidase-conjugated anti-rabbit secondary antibody.
   **(A)** As shown APG1181 specifically recognises an epitope that encodes the N-terminal aa13-19 of human CD95L. Using permutated peptides (8-11 figure 8) for fine mapping of the epitope reveals the Y(tyrosine) in position 13 is absolutely required for defining the specificity of the respective antibody APG1181/24-8. The W(tryptophan) in position 14 and the A(alanin) in position 19, respectively, do only have a marginal influence on the binding specificity (epitope) of the antibody.
   **(B)** A different antibody (clone 39-9) that recognises a similar epitope but does not have the absolute requirement for the "Y" in position 13, does surprisingly show no specific reactivity towards CD95L in IHC-based analysis.

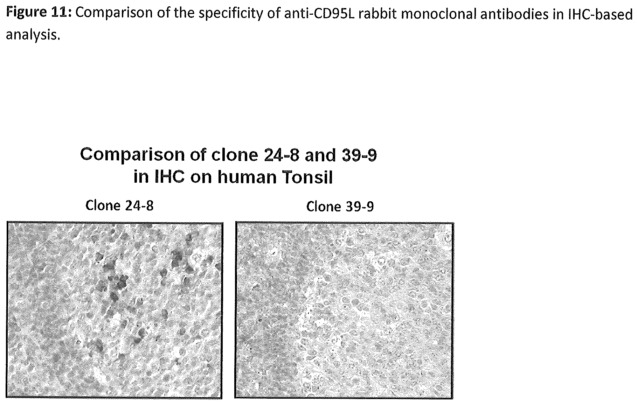

**Figure 11****:** Comparison of the specificity of anti-CD95L rabbit monoclonal antibodies in IHC-based analysis.
   Purified antibodies from clone 24-8 and clone 39-9 were analysed for their ability to detect CD95L in a subset of plasma cells on human tosil sections. Although both antibodies share a similar epitope, clone 39-9 does surprisingly show no reactivity towards CD95L in IHC-based analysis. Based on this result it can be concluded that the "Y" in position 13 is a prerequisite for IHC reactivity of clone 24-8.

### Example 1

### APG101 (CD95 ligand inhibitor) for treatment of cancer: a phase II study

The phase II study was a randomised, open-label, multi-centre study of weekly APG101 plus re-irradiation (APG101+RT group, test group) versus re-irradiation alone (RT group, control group) in the treatment of patients with first or second relapse or progression of glioblastoma. In total, 91 patients were included in the study. The ITT population consisted of 84 patients, 58 in the APG101+RT group and 26 in the RT group. Male and female patients with glioblastoma at first or second relapse either not being eligible for tumor resection or having macroscopic residual tumor after resection were candidates for this study. Eligible patients must have failed standard treatment that must have included radiotherapy (60Gy) and temozolomide and had to be a candidate for re-irradiation. Patients included in the study received either re-irradiation alone or re-irradiation plus 400 mg APG101 administered as a 30 min i.v. infusion once every week until progression.

Archived tumor tissue for histological analyses was available from 81 patients (of the ITT population) to confirm glioblastoma diagnosis. For the central histology review, biopsy or surgery material embedded in paraffin must have been available for central review in order to confirm the primary diagnosis of glioblastoma. All samples that were obtained were archived samples originating from the initial surgery and disease diagnosis or pre-study surgical resections. Following registration of the patient, a paraffin embedded tumor sample was collected by the study centre. Tissue sections were prepared from these tumor samples to assess various "markers" including CD95L using immunohistochemical staining (IHC).

The glioblastoma multiforme (GBM) patients participating in this clinical study were randomized into the control group and a test group.

### Results

**Figure 1** describes the survival rate of the patients of the control group and the test group (overall survival rate, OS). The figure indicates that the patients of the test group receiving APG101 and radiation therapy have an improved survival rate compared with the control group receiving radiation therapy only (see **Table 1**).

**Table 1: overall survival in all patients**

| | **RT only (OS rates)** | **APG+RT (OS rates)** |
|---|---|---|
| 6m | 77% | 90% |
| 12m | 50% | 50% |
| 18m | 23% | 34% |
| 24m | 7.3% | 22% |

Sections of GBM tumor samples of the control group patients and the test group patients were analysed for the presence of CD95 ligand (CD95L) by immunohistolochemical staining **(see Example 2**).

It was surprisingly found that the level and distribution of CD95L expression strongly differed among the patients. About one third of patients exhibited a strong positive CD95L expression in the tumor tissue. Yet another third of patients exhibited a CD95L negative phenotype. The remaining patients showed a slight expression of CD95L. This phenotype could be termed "low positive" phenotype.

In the test group patients receiving APG101 on top of radiation therapy (N=58), 18 patients exhibited the strong positive CD95L expression phenotype in the tumor tissue. 18 patients exhibited the CD95L negative phenotype. In the control group (N=26), 10 patients showed the strong positive CD95L phenotype, and 9 patients showed the CD95L negative phenotype (**Table 2**).

**Table 2: Overall survival times of the test group (APG101 on top of radiation therapy, "APG+RT") and in the control group (radiation therapy only, "RT only"). N: number of patients. The median is given in days. "95% (-/+)" indicates the 95% confidence interval.**

| | CD95Lpos. | | | CD95L neg. | | |
|---|---|---|---|---|---|---|
| | N | 95% (-/+) | median | N | 95% (-/+) | median |
| APG+RT | 18 | 200/500 | 350 | 18* | 140/670 | 410 |
| RT only | 10 | 59 / 440 | 250 | 9 | 190/740 | 460 |

| | | | | | | |
|---|---|---|---|---|---|---|
| * 3 patients censored in the APG group (still alive) | | | | | | |

Surprisingly, the overall survival time of the control group GBM patients was negatively correlated with the level of CD95L expression. The patients showing the strong CD95L positive phenotype of GBM had a worse prognosis than patients with CD95L negative GBM. CD95L positive patients survived 250 days, wherein CD95L negative patients survived 460 days (see **Table 2).**

In the test group, the patients showing the strong CD95L positive phenotype of GBM benefited from the treatment. CD95L positive patients survived 350 days.

The Kaplan-Meyer plot of **Figure 2** compares the survival data obtained in CD95L positive patients in the control group and the test group. Upon treatment with APG101, the curve is shifted to larger survival times. This means that patients showing the CD95L positive phenotype of GBM benefited from the administration of APG101. The median overall survival time increased from 250 days (control group) to 350 days.

In the test group, median overall survival in CD95 negative patients was 410 days. In comparison to the control group patients showing the negative CD95L phenotype, the median overall survival decreased from 460 to 410 days upon treatment with APG101. The data are described in the Kaplan-Meyer plot of **Figure 3****.** This figure indicates that the curves of the test group and the control group are largely overlapping and not statistically significant.

### Summary

This example demonstrates that the expression of CD95L in glioblastoma is associated with a reduced survival of the patients. This means that CD95L is suitable as a prognostic factors in glioblastoma. This is important as glioblastoma expresses CD95L to a variable extent. Therefore, the present invention enables a diagnostic differentiation of glioblastoma by expression of CD95L. This leads to a new diagnostic differentiation of glioblastoma in CD95L positive glioblastoma and CD95L negative glioblastoma.

Furthermore, this example surprisingly demonstrates that a CD95L inhibitor, for example APG101, can improve the survival of glioblastoma patients suffering from a glioblastoma expressing CD95L. In contrast, patients suffering from a glioblastoma which does not express CD95L, do not benefit from the treatment with a CD95L inhibitor. This example enables a therapeutic strategy comprising determining the CD95L expression in a glioblastoma sample, and treating the patient if CD95L is expressed. This strategy is advantageous, because the CD95L inhibitor can be administered to those patients in which a therapeutic success can be expected. This is not a disadvantage for patients suffering from a glioblastoma not expressing CD95L, because these patients probably do not benefit from a treatment with a CD95L inhibitor.

### Example 2

### Immunohistochemical determination of CD95L

CD95L can be determined in a tissue section (for example a tumor tissue section) by performing the following procedure:
- Tissue samples were fixed in 4% buffered formaldehyde and embedded in paraffin.
- 3 µm thick sections were dewoxed:
   1. Xylol 10 min.
   2. Xylol 10 min.
   3. Xylol 10 min.
   1. Ethanol 100% 5 min.
   2. Ethanol 100% 5 min.
      Ethanol 96% 5 min.
      Ethanol 90% 5 min.
      Ethanol 70% 5 min.
   1. H₂O dest. 1 min
   2. H₂O dest. 5 min.
- High temperature antigen demasking procedures:
   Citrat pH 6.0 (Target Retrieval Solution), 99°C, (DAKO, Cat.No. S1699), 25 min.
- Cooling 15 min at room temperature
- Washing 2x 5 min in PBS
- *For tissue with high endogenous biotin: Biotin-Block (DAKO, Biotin Blocking System, X0590), for example for samples obtained from pancreas, liver, kidney,*
   → *Avidin 10 min. room temperature (RT)*
   → *Rinse in PBS 1x 5 min*
   → *Biotin 10 min. RT*
   → *Rinse in PBS 1x 5 min*
- To block unspecific antibody binding, sections were incubated with blocking solution 1 (PBS, BSA 20 mg/ml; Serva, Germany, Cat.No.11924), human IgG 1 mg/ml, (Gammunex 10%, Talecris) for 20 min.
- 1^{st} Antibody diluted in blocking solution 1
   → Anti CD95 aAPG101 Rabbit Fc depleted 2µg/ml
   → Anti CD95L polyclonal rabbit (Dianova, Cat.No. DLN-14047) 1:100
   → Rabbit IgG isotype control (Invitrogen (Zytomed), Cat.No. 08-6199)
- Incubation 60 minutes at room temperature.
- Washing 2x 5 min in PBS
- Blocking with blocking solution 2 for 20 minutes. Blocking with blocking solution 2 = blocking with blocking solution 1 + 20% normal goat serum, Dianova, Cat.No. 005-000-121.
- 2^{ndary} antibody diluted in blocking solution 2, for example Goat F(ab')₂ Anti_Rabbit IgG (H+L chain) biotinylated, (SouthernBiotech, Cat.No. 4052-08) 1:100, Incubation 30 minutes at room temperature
- Washing 2x 5 min in PBS
- Streptavidin-alkaline phosphatase (Concentrated AP label, BioGenex, Cat:No. HK321-UK), diluted in blocking solution 1, incubation 30 minutes at room temperature
- Washing 2x 5 min in PBS
- Incubation with alkaline phosphatase substrate (DAKO Liquid Permanent Red, DakoCytomation GmbH, Glostrup, Denmark, Cat.No. K0640)
- Counterstaining with hematoxylin (Merck, Mayers Hämalaunlösung Cat.No. 1.09249.0500)
- Determination of the number of stained cells with respect to the total number of cells (% CD95L positive cells), or/and the size of the stained area with respect to the total area (% CD95L positive area). If tumor tissue is examined: determination of the number of stained tumor cells with respect to the total number of tumor cells (% CD95L positive tumor cells), or/and the size of the stained area of tumor tissue with respect to the total area of tumor tissue (% CD95L positive area of tumor tissue). Non-tumor tissue is disregarded.

### Example 3

### Evaluation of CD95L immunohistochemically stained slides

### 1. General aspects

All CD95L-stained slides (see Example 2) were evaluated by a single board-certified neuropathologist with extensive experience in the field of neurooncology (C.H.). The whole evaluation was performed slide-by-slide in a single session. The longest intermission in the whole evaluation process was not longer than 60 min in between. The neuropathologist was blinded for the clinical data of the patients. The same neuropathologist had generated the reference histology diagnosis based on H&E- and silver staining combined with Mib1-, GFAP- and IDH1 R132H immunohistochemistry before. However, at the time of CD95L-evaluation these diagnosis-related slides were not available. The neuropathologist only had personal notices available about the diagnosis. Furthermore, the neuropathologist had evaluated the MGMT status and results from this analysis were known when the CD95L slides were assessed.

### 2. Generation of calibration figures

The CD95L immunohistochemistry staining was established by the company Apogenix. The evaluating neuropathologist (C.H.) was not involved in the technical aspects of the stainings, he was blinded regarding the protocols that were applied. After finalization of the CD95L immunohistochemistry establishing process a small number of CD95L-stained slides were committed to the neuropathologist to check for the quality. The neuropathologist confirmed that the applied immunohistochemistry allows high quality results and agreed to define this protocol to be the standard for further CD95L stainings.
Based on these slides that were generated for quality assessment calibration figures were created that allowed comparison of CD95L immunohistochemistry intensities. Pictures were taken on a Carl Zeiss AxioPlan2 microscope with an AxioCamHR digital camera using the software AxioVision 4.8. The unmodified pictures were directly incorporated in CorelDraw v14.0 and arranged. Two pictures from different samples of similar CD95L immunohistochemistry intensities were set in one line. In summary, 2 lines of pictures representing the CD95L immunohistochemistry intensities "CD95L positive (high)" or "CD95L negative (low)" were generated (see **Figure 4**).

### 3. Overview and tissue selection

In a first step the slide was scanned under the microscope (Olympus BX46) with low resolution to evaluate the amount of solid tumor tissue. Areas of normal brain parenchyma and areas of tumor infiltration were excluded from further analysis. Tumor tissue was selected for further evaluation if the following criteria were fulfilled:
- Tumor tissue that appeared to be vital without signs for hypoxic damage.
- Tumor tissue without resection-induced fresh bleedings.
- Tumor tissue without signs of cutting-induced artifacts.

### 4. Single slide evaluation - criterion 'intensity'

The suitable tumor tissue of each slide was evaluated regarding the CD95L positive or CD95L negative staining intensities. The CD95L calibration figure was used to standardize the evaluation. Because most tumors showed different staining intensities in separate areas the percentage of these summed areas were counted. Each tumor was assigned a specific value in percent representing the area showing CD95L positive or CD95L negative staining intensity:

| Intensity | Percentage |
|---|---|
| Absent (CD95L negative) | < 2 % |
| CD95L positive | > 5% |

Finally, the results were noticed in central pathology review form.

### 5. Single slide evaluation - criterion 'distribution pattern'

A glial tumor (for example a glioblastoma) is composed of tumor cells and a fibrillary matrix of tumor cell processes. Using a light microscope these processes usually cannot be assigned to a particular tumor cell. If CD95L antibody binding was predominately seen in such cellular compartment of the matrix the staining pattern was defined to be 'diffuse'. If instead clearly tumor cells were CD95L-labeled the staining pattern was defined to be 'focal'. If essentially no CD95L labeling was observed in the tumor tissue, or the CD95L positive area was below 2% of tumor tissue, the distribution pattern was not evaluated. Finally, the results were noticed in central pathology review form.

### 6. Reporting of CD95L results

Results from the CD95L evaluation were noticed in the central pathology review form (see above). The form was dated and signed by the responsible neuropathologist.

### 7. Generation of a specific CD95L-antibody suited for IHC-based analysis

CD95L specific rabbit monoclonal antibodies employing immunization of rabbits with a synthetic N-terminal peptide, encoding the first 21aa of human CD95L (CD95L_NT 1-21: MQQPFNYPYPQIYWVDSSASS) were developed. For the selection of an appropriate antibody suited for the detection of CD95L in an IHC-based assay, a thorough characterization of candidate anti-CD95L rabbit monoclonal antibodies including IHC-analysis was performed. The screening process identified numerous antibodies that specifically recognised the N-terminal peptide used for immunization. However, one rabbit monoclonal antibody "clone 24-8" was in particular suited for the detection of CD95L in IHC-based analysis.

To ensure constant antibody supply recombinant expression of the antibody derived from clone 24-8 was employed in HEK cells. The recombinant antibody derived from clone 24-8 is based on the sequence of the respective IgG heavy and light chain and was designated APG1181. The specificity of the recombinant antibody is identical to the antibody derived from the hybridoma clone 24-8 (see figure 5 and figure 7). The specificity of the antibody was characterized by ELISA, IHC-analysis and mapping of the specific epitope.

## Claims

1. A method for diagnosing glioblastoma, comprising
(a) determining the expression of CD95L in a glioblastoma sample, and
(b) classifying the glioblastoma according the level of CD95L expression, wherein the glioblastoma is classified by the level of CD95L expression into a CD95L positive glioblastoma or a CD95L negative glioblastoma, wherein the glioblastoma is regarded as CD95L positive if at least 5% of the cells in the sample express CD95L and/or if CD95L can be detected on at least 5% of the area of tumor tissue in a glioblastoma tissue sample.

2. The method according to claim 1, wherein the expression of CD95L in the glioblastoma sample is determined by contacting the sample with an agent specifically binding to CD95L, wherein the agent
(i) comprises a fusion protein comprising at least an extracellular CD95 domain or a functional fragment thereof and at least a Fc domain or a functional fragment thereof, wherein the fusion protein is preferably selected from APG101, polypeptides having at least 70% identity to APG101 and functional fragments of APG101 and/or
(ii) is an anti-CD95L specific antibody or a CD95L recognising fragment thereof, which preferably binds to an intracellular epitope of CD95L, preferably comprising the amino acid Y in N-terminal position 13 of human CD95L, in particular wherein the anti-CD95L-specific antibody or CD95L-recognising fragment thereof recognises an epitope that encodes the N-terminal amino acids 13-19 of human CD95L.

3. A method of prognosing the overall survival time or/and the relapse-free survival time in a glioblastoma patient, said method comprising
(a) determining CD95L expression in a glioblastoma sample,
(b) classifying the glioblastoma by the level of CD95L expression into a CD95L positive glioblastoma or a CD95L negative glioblastoma, and
(c) prognosing the survival time or/and the relapse-free survival time of the patient by the level of CD95L expression, wherein the CD95L expression is negatively correlated with the survival time of the patient.

4. The method according to claim 3, wherein the CD95L expression of step (a) is determined by
(i) a fusion protein comprising at least an extracellular CD95 domain or a functional fragment thereof and at least a Fc domain or a functional fragment thereof, wherein the fusion protein is selected from APG101, polypeptides having at least 70% identity to APG101 and functional fragments of APG101, and/or
(ii) an anti-CD95L specific antibody or a CD95L recognising fragment thereof, which preferably binds to an intracellular epitope of CD95L, preferably comprising the amino acid Y in N-terminal position 13 of human CD95L, in particular wherein the anti-CD95L-specific antibody or CD95L-recognising fragment thereof recognises an epitope that encodes the N-terminal amino acids 13-19 of human CD95L.

5. A CD95L inhibitor for use in the treatment of glioblastoma, wherein the CD95L inhibitor comprises a fusion protein comprising at least an extracellular CD95 domain or a functional fragment thereof and at least a Fc domain or a functional fragment thereof, and wherein the glioblastoma is a CD95L positive glioblastoma that has been classified as a CD95L positive glioblastoma according to the method of any one of claims 1 and 2.

## Patentansprüche

1. Verfahren zum Diagnostizieren von Glioblastom, umfassend
(a) Bestimmen der Expression von CD95L in einer Glioblastom-Probe und
(b) Klassifizieren des Glioblastoms nach dem Level an CD95L Expression, wobei das Glioblastom durch das Level an CD95L Expression in ein CD95L positives Glioblastom oder ein CD95L negatives Glioblastom klassifiziert wird, wobei das Glioblastom als CD95L positiv angesehen wird wenn wenigstens 5% der Zellen in der Probe CD95L exprimieren und/oder wenn CD95L auf wenigstens 5% der Tumorgewebefläche in einer Glioblastom-Gewebeprobe festgestellt werden kann.

2. Verfahren nach Anspruch 1, wobei die Expression von CD95L in der Glioblastom-Probe durch ein in Kontakt bringen der Probe mit einem Mittel, das spezifisch an CD95L bindet, bestimmt wird, wobei das Mittel
(i) ein Fusionsprotein umfasst, das wenigstens eine extrazelluläre CD95 Domäne oder ein funktionelles Fragment davon und wenigstens eine Fc Domäne oder ein funktionelles Fragment davon umfasst, wobei das Fusionsprotein bevorzugt aus APG101, Polypeptiden, die wenigstens 70% Identität zu APG101 aufweisen, und funktionellen Fragmenten von APG101 ausgewählt ist und/oder
(ii) ein anti-CD95L spezifischer Antikörper oder ein CD95L erkennendes Fragment davon ist, welches bevorzugt an ein intrazelluläres Epitop von CD95L bindet, bevorzugt umfassend die Aminosäure Y in N-terminaler Position 13 von humanem CD95L, insbesondere wobei der anti-CD95L-spezifische Antikörper oder das CD95L-erkennende Fragment davon ein Epitop erkennt, das für die N-terminalen Aminosäuren 13-19 von humanem CD95L kodiert.

3. Verfahren zum Prognostizieren der Gesamtüberlebensdauer oder/und der Rückfall-freien Überlebensdauer eines Glioblastom-Patienten, wobei das Verfahren umfasst
(a) Bestimmen der CD95L Expression in einer Glioblastom-Probe,
(b) Klassifizieren des Glioblastoms durch das Level an CD95L Expression in ein CD95L positives Glioblastom oder ein CD95L negatives Glioblastom und
(c) Prognostizieren der Überlebensdauer oder/und der Rückfall-freien Überlebensdauer des Patienten durch das Level an CD95L Expression, wobei die CD95L Expression mit der Überlebensdauer des Patienten negativ korreliert ist.

4. Verfahren nach Anspruch 3, wobei die CD95L Expression von Schritt (a) bestimmt wird durch
(i) ein Fusionsprotein, das wenigstens eine extrazelluläre CD95 Domäne oder ein funktionelles Fragment davon und wenigstens eine Fc Domäne oder ein funktionelles Fragment davon umfasst, wobei das Fusionsprotein aus APG101, Polypeptiden, die wenigstens 70% Identität zu APG101 aufweisen, und funktionellen Fragmenten von APG101 ausgewählt ist und/oder
(ii) einen anti-CD95L spezifischen Antikörper oder ein CD95L erkennendes Fragment davon, welches bevorzugt an ein intrazelluläres Epitop von CD95L bindet, bevorzugt umfassend die Aminosäure Y in N-terminaler Position 13 von humanem CD95L, insbesondere wobei der anti-CD95L-spezifische Antikörper oder das CD95L-erkennende Fragment davon ein Epitop erkennt, das für die N-terminalen Aminosäuren 13-19 von humanem CD95L kodiert.

5. CD95L Inhibitor zur Verwendung in der Behandlung von Glioblastom, wobei der CD95L Inhibitor ein Fusionsprotein umfasst, das wenigstens eine extrazelluläre CD95 Domäne oder ein funktionelles Fragment davon und wenigstens eine Fc Domäne oder ein funktionelles Fragment davon umfasst und wobei das Glioblastom ein CD95L positives Glioblastom ist, das nach dem Verfahren von einem der Ansprüche 1 und 2 als ein CD95L positives Glioblastom klassifiziert worden ist.

## Revendications

1. Procédé de diagnostic du glioblastome, comprenant
(a) la détermination de l'expression du CD95L dans un échantillon de glioblastome, et
(b) la classification du glioblastome selon le taux d'expression du CD95L, dans lequel le glioblastome est classifié par le taux d'expression du CD95L en un glioblastome positif au CD95L ou un glioblastome négatif au CD95L, dans lequel le glioblastome est considéré comme positif au CD95L si au moins 5 % des cellules dans l'échantillon expriment CD95L et/ou si CD95L peut être détecté sur au moins 5 % de la superficie du tissu tumoral dans un échantillon de tissu du glioblastome.

2. Procédé selon la revendication 1, dans lequel l'expression du CD95L dans l'échantillon de glioblastome est déterminée par mise en contact de l'échantillon avec un agent se liant spécifiquement au CD95L, dans lequel l'agent
(i) comprend une protéine de fusion comprenant au moins un domaine extracellulaire du CD95 ou un fragment fonctionnel de celui-ci et au moins un domaine Fc ou un fragment fonctionnel de celui-ci, dans lequel la protéine de fusion est de préférence choisie parmi APG101, des polypeptides ayant au moins 70 % d'identité avec APG101 et des fragments fonctionnels d'APG101 et/ou
(ii) est un anticorps spécifique anti-CD95L ou un fragment de celui-ci reconnaissant CD95L, qui se lie de préférence à un épitope intracellulaire du CD95L, de préférence comprenant l'acide aminé Y dans la position N-terminale 13 du CD95L humain, en particulier dans lequel l'anticorps spécifique anti-CD95L ou le fragment de celui-ci reconnaissant CD95L reconnaît un épitope qui code les acides aminés N-terminaux 13 à 19 du CD95L humain.

3. Procédé de pronostic du temps de survie global et/ou du temps de survie exempt de rechute chez un patient atteint du glioblastome, ledit procédé comprenant
(a) la détermination de l'expression du CD95L dans un échantillon de glioblastome,
(b) la classification du glioblastome par le taux d'expression du CD95L en un glioblastome positif au CD95L ou un glioblastome négatif au CD95L, et
(c) le pronostic du temps de survie et/ou du temps de survie exempt de rechute du patient par le taux d'expression du CD95L, dans lequel l'expression du CD95L est corrélée négativement au temps de survie du patient.

4. Procédé selon la revendication 3, dans lequel l'expression du CD95L de l'étape (a) est déterminée par
(i) une protéine de fusion comprenant au moins un domaine extracellulaire du CD95 ou un fragment fonctionnel de celui-ci et au moins un domaine Fc ou un fragment fonctionnel de celui-ci, dans lequel la protéine de fusion est de préférence choisie parmi APG101, des polypeptides ayant au moins 70 % d'identité avec APG101 et des fragments fonctionnels d'APG101, et/ou
(ii) un anticorps spécifique anti-CD95L ou un fragment de celui-ci reconnaissant CD95L, qui se lie de préférence à un épitope intracellulaire du CD95L, de préférence comprenant l'acide aminé Y dans la position N-terminale 13 du CD95L humain, en particulier dans lequel l'anticorps spécifique anti-CD95L ou le fragment de celui-ci reconnaissant CD95L reconnaît un épitope qui code les acides aminés N-terminaux 13 à 19 du CD95L humain.

5. Inhibiteur du CD95L pour utilisation dans le traitement d'un glioblastome, dans lequel l'inhibiteur du CD95L comprend une protéine de fusion comprenant au moins un domaine extracellulaire du CD95 ou un fragment fonctionnel de celui-ci et au moins un domaine Fc ou un fragment fonctionnel de celui-ci, et dans lequel le glioblastome est un glioblastome positif au CD95L qui a été classifié comme un glioblastome positif au CD95L selon le procédé de l'une quelconque des revendications 1 et 2.


### SEQUENCE LISTING

<110> Apogenix GmbH
<120> Method of diagnosing cancer
<130> 54306P WO
<160> 1
<170> PatentIn version 3.5
<210> 1
   <211> 400
   <212> PRT
   <213> Artificial Sequence
<220>
   <223> Recombinant fusion protein consisting of human CD95 extracellular domain with human IgG1 FC-part to its C-terminus
<220>
   <221> SIGNAL
   <222> (1)..(16)
   <223> Variable cleavage sites
<220>
   <221> SIGNAL
   <222> (1)..(20)
   <223> Variable cleavage sites
<220>
   <221> SIGNAL
   <222> (1)..(25)
   <223> Variable cleavage sites
<220>
   <221> DOMAIN
   <222> (26)..(172)
   <223> Human CD95 extracellular domain
<220>
   <221> MOD_RES
   <222> (26)..(26)
   <223> PYRROLIDONE CARBOXYLIC ACID
<220>
   <221> DISULFID
   <222> (59)..(73)
<220>
   <221> DISULFID
   <222> (63).. (82)
<220>
   <221> DISULFID
   <222> (85).. (101)
<220>
   <221> DISULFID
   <222> (104)..(119)
<220>
   <221> DISULFID
   <222> (107)..(127)
<220>
   <221> CARBOHYD
   <222> (118)..(118)
   <223> N-linked Glycosylation at Position N118
<220>
   <221> DISULFID
   <222> (129)..(143)
<220>
   <221> DISULFID
   <222> (135).. (140)
<220>
   <221> CARBOHYD
   <222> (136)..(136)
   <223> N-linked Glycosylation at Position N136
<220>
   <221> DISULFID
   <222> (146)..(157)
<220>
   <221> DISULFID
   <222> (149)..(165)
<220>
   <221> DOMAIN
   <222> (172)..(400)
   <223> Human IgGl-FC domain (one amino acid overlap with CD95 domain)
<220>
   <221> DISULFID
   <222> (173)..(173)
   <223> Interchain cystine forming residue.
<220>
   <221> DISULFID
   <222> (179)..(179)
   <223> Interchain cystine forming residue.
<220>
   <221> DISULFID
   <222> (182)..(182)
   <223> Interchain cystine forming residue
<220>
   <221> DISULFID
   <222> (214)..(274)
<220>
   <221> CARBOHYD
   <222> (250)..(250)
   <223> N-linked Glycosylation at Position N250
<220>
   <221> DISULFID
   <222> (320) .. (378)
<400> 1
   Met Val Gly Ile Trp Thr Leu Leu Pro Leu Val Leu Thr Ser Val Ala
   1               5                   10                  15
   Arg Leu Ser Ser Lys Ser Val Asn Ala Gln Val Thr Asp Ile Asn Ser
               20                  25                  30
   Lys Gly Leu Glu Leu Arg Lys Thr Val Thr Thr Val Glu Thr Gln Asn
           35                  40                  45
   Leu Glu Gly Leu His His Asp Gly Gln Phe Cys His Lys Pro Cys Pro
       50                  55                  60
   Pro Gly Glu Arg Lys Ala Arg Asp Cys Thr Val Asn Gly Asp Glu Pro
   65                  70                  75                  80
   Asp Cys Val Pro Cys Gln Glu Gly Lys Glu Tyr Thr Asp Lys Ala His
                   85                  90                  95
   Phe Ser Ser Lys Cys Arg Arg Cys Arg Leu Cys Asp Glu Gly His Gly
               100                 105                 110
   Leu Glu Val Glu Ile Asn Cys Thr Arg Thr Gln Asn Thr Lys Cys Arg
           115                 120                 125
   Cys Lys Pro Asn Phe Phe Cys Asn Ser Thr Val Cys Glu His Cys Asp
       130                 135                 140
   Pro Cys Thr Lys Cys Glu His Gly Ile Ile Lys Glu Cys Thr Leu Thr
   145                 150                 155                 160
   Ser Asn Thr Lys Cys Lys Glu Glu Gly Ser Arg Ser Cys Asp Lys Thr
                   165                 170                 175
   His Thr Cys Pro Pro Cys Pro Ala Pro Glu Leu Leu Gly Gly Pro Ser
               180                 185                 190
   Val Phe Leu Phe Pro Pro Lys Pro Lys Asp Thr Leu Met Ile Ser Arg
           195                 200                 205
   Thr Pro Glu Val Thr Cys Val Val Val Asp Val Ser His Glu Asp Pro
       210                 215                 220
   Glu Val Lys Phe Asn Trp Tyr Val Asp Gly Val Glu Val His Asn Ala
   225                 230                 235                 240
   Lys Thr Lys Pro Arg Glu Glu Gln Tyr Asn Ser Thr Tyr Arg Val Val
                   245                 250                 255
   Ser Val Leu Thr Val Leu His Gln Asp Trp Leu Asn Gly Lys Glu Tyr
               260                 265                 270
   Lys Cys Lys Val Ser Asn Lys Ala Leu Pro Ala Pro Ile Glu Lys Thr
           275                 280                 285
   Ile Ser Lys Ala Lys Gly Gln Pro Arg Glu Pro Gln Val Tyr Thr Leu
       290                 295                 300
   Pro Pro Ser Arg Glu Glu Met Thr Lys Asn Gln Val Ser Leu Thr Cys
   305                 310                 315                 320
   Leu Val Lys Gly Phe Tyr Pro Ser Asp Ile Ala Val Glu Trp Glu Ser
                   325                 330                 335
   Asn Gly Gln Pro Glu Asn Asn Tyr Lys Thr Thr Pro Pro Val Leu Asp
               340                 345                 350
   Ser Asp Gly Ser Phe Phe Leu Tyr Ser Lys Leu Thr Val Asp Lys Ser
           355                 360                 365
   Arg Trp Gln Gln Gly Asn Val Phe Ser Cys Ser Val Met His Glu Ala
       370                 375                 380
   Leu His Asn His Tyr Thr Gln Lys Ser Leu Ser Leu Ser Pro Gly Lys
   385                 390                 395                 400